# EUROPEAN PATENT SPECIFICATION

(11) **EP 1 370 271 B1**
(45) Date of publication and mention of the grant of the patent: **05.10.2005**
(21) Application number: 02702393.6
(22) Date of filing: 26.02.2002
(51) Int. Cl.: A61K 35/78

(54) **A PROCESS FOR THE MANUFACTURE OF A HERBAL COMPOSITION COMPRISING A MATRINE**
PROZESS ZUR HERSTELLUNG EINES MATRIN ENTHALTENDEN PFLANZENEXTRAKTS
PROCEDE DE PRODUCTION D'UNE COMPOSITION VEGETALE COMPRENANT UNE MATRINE

(30) Priority: 26.02.2001 US 793251
(43) Date of publication of application: 17.12.2003
(73) Proprietor: Global Cancer Strategies Ltd., British Columbia V6E 4M2 (CA)
(72) Inventor: TZE, John, Wah, deceased (CA); LIN, Peizhong, Chaoyang District, Beijing 100021 (CN); LAM, Stephen, Vancouver, British Columbia V6T 2E3 (CA); TAI, Joseph, North Vancouver, British Columbia (CA); SMIT, Frisi, Hobbe, NL-3524 AS Utrecht (NL); VAN HELVOORT, Adrianus, Lambertus, Bertholdus, NL-6708 CP Wageningen (NL); HAGEMAN, Robert, Johan,Joseph., NL-6705 CT Wageningen (NL); TZE, Theresa, P., Chiang, Vancouver, BC V6M 2X4 (CA)
(74) Representative: van Westenbrugge, Andries
(86) International application number: PCT/EP2002/002350
(87) International publication number: WO 2002/067955

(56) References cited:
- WO-A-01/03749

## Description

### TECHNICAL FIELD OF THE INVENTION

The present invention is concerned with a process for the manufacture of a herbal composition. More particularly the present invention relates to a process that comprises subjecting a plurality of herbs to extraction with an aqueous solvent so as to produce at least 2 herbal extracts which are mixed and subsequently dried and ground to produce a granulate.

The herbal composition according to the present invention may advantageously be used in a method of treating or preventing cancer, *H. pylori* infections, chronic inflammatory conditions, cardiovascular diseases or cerebral vascular diseases in mammals.

### BACKGROUND OF THE INVENTION

Nowadays a huge number of different drugs is available for combating a large variety of cancers. Most of these drugs are composed of individual or combinations of chemicals that do not occur in nature. While some of these chemicals are effective, many have serious side effects which prevent their long-term and/or recurrent use.

Traditionally, the focus of cancer research has been in developing therapies and treatment for patients already afflicted with cancer. However, over the last few decades, new insights in the development of cancer as a disease have been gained. It is now understood that cancer is not the result of a single initiation event, but of a gradual, multi-step process characterised by a period of several years between the initiation event and the onset of invasive or metastatic disease. In general, the process of carcinogenesis can be divided into three phases: initiation, promotion and progression. In initiation, a fixed genetic mutation results from the interaction of a carcinogen with DNA. The extent of the molecular change depends on a number of factors including the nature of the nature of the carcinogen, the rate and type of carcinogenic metabolism and the response of the DNA repair system. The next phase, promotion, may occur over extended periods of time and is characterised by the proliferation of the altered cells. This phase may be affected by agents that alter growth rates. During the final phase, progression, genetic and phenotypic changes occur which ultimately cause the development of premalignant lesions into invasive cancer.

The multi-step nature of carcinogenesis suggests the possibility of intervention at a precancerous state. This is the basis of chemoprevention, which refers to the use of natural or synthetic agents to prevent the development of cancer, either by blocking the DNA damage that initiates the carcinogenesis process or by arresting/regressing existing pre-malignant lesions.

Since the mid-1950's, research has been directed at finding components with potential chemopreventive properties. The search for these agents has demonstrated an unique challenge. Chemopreventive agents must have low toxicity and be relatively free of side effects because they are intended for administration to healthy people over long periods of time. This is in direct contrast with chemotherapy drugs. Chemotherapeutic drugs are chosen for their ability to kill tumor cells, but because they are also toxic to healthy cells, they usually cause harmful side effects.

One of the major sources of potential chemopreventive agents is plants. For example, consumption of cruciferous vegetables, such as broccoli, cauliflower and cabbage is associated with a lower risk of various cancers. Fruits and vegetables contain a number of potentially active chemopreventive compounds, such carotenoids, dithiolthiones and isothiocyanates. A variety of Chinese herbs have been used for centuries to treat different diseases. Although some of these herbs have been used to treat patients with cancer, they are not considered to be disease specific. Herbs such as *Sophora tonkinensis, Polygonum bistorta, Prunella vulgaris, Sonchus brachyotus, Dictamnus dasycarpus Turcz* and *Dioscorea bulbifera* comprise components that have been shown to display anti-proliferative effects in certain *in vivo* assays. A herbal composition containing one or more of the aforementioned herbs is known as ZSP or Zeng Sheng Ping; however, the exact formulation of the composition is not known. In traditional Chinese herbal medicine, it is common practice to substitute selected herbs with other herbs depending upon the symptoms of an individual patient and/or local availability of certain herbs.

The aforementioned herbs are traditionally dried and ground into a powder so they can be dissolved into hot water and be administered as a potion. Research into the constituents of herbs with medicinal properties has enabled the identification of substances that are believed to be at least partly responsible for these medicinal properties. This has also stimulated the development of effective isolation techniques for obtaining these active principles from said herbs in concentrated form.

An example of a substance that has been found in herbs and which is believed to have medicinal properties, particularly as a chemopreventive agent, is matrine. Matrine is a substance that is found, for instance, in the herbs *Sophora tonkinensis (Sophora subprostrata), Belamcanda chinensis (L) DC, Scrophyuloaria ningpoensis Hemsl., Isatis tinctoria L., Isalis indignotica Fort.* and *Baphocacanthus cusia Sophora tonkinensis (Sophora subprostrata), Belamcanda chinensis (L) DC, Scrophyuloaria ningpoensis Hemsl., Isatis tinctoria L., Isatis indignotica Fort.* and *Baphocacanthus cusia Bremek.* Often matrine is found in these herbs together with oxymatrine. Oxymatrine is a matrine precursor which is converted in the gastrointestinal tract to form matrine.

Another substance that is found in herbs and that is deemed to have chemopreventive and therapeutic properties is dictamnine. Dictamnine is a substance found in herbs belonging to the species *Dictamnus*, especially *Dictamnus dasycarpus Turcz.*

It is well known in the art to produce a herbal composition by combining one or more plant materials and subjecting the resulting combination of plant materials to an extraction so as to produce an isolate. The objective of such methods is to isolate the active principles, e.g. to separate these from toxic or otherwise undesired components contained in the plant material, and to subsequently remove most of the extraction solvent so as to obtain the active principles in concentrated form. Premixing of the plant materials in this fashion, prior to extraction, is often deemed to be a more efficient practice than separately extracting each individual plant material followed by admixture of the resulting extracts.

### SUMMARY OF THE INVENTION

The inventors have unexpectedly found that if matrine containing plant material is combined with certain herb varieties, in particular herbs belonging to the species *Dictamnus,* to produce an aqueous extract, the recovery of matrine in the resulting extract is very low. It was discovered that the beneficial properties of both the matrine containing plant material and the *Dictamnus* derived material can be retained in a single composition if these 2 materials are extracted separately and combined thereafter.

In addition it was found that the following herbs may also interfere with the recovery of matrine from plant materials: *Polygonum bistorta, Prunella vulgaris, Sonchus brachyotus* and *Dioscorea bulbifera*. Such interference is particularly pronounced in the presence of plant material from the species *Dictamnus*.

Consequently, the present invention aims to present a process for the preparation of a herbal composition containing a significant amount of matrine, which herbal composition is obtained by subjecting a number of plant materials, including at least one matrine containing plant material and a plant material derived from a plant belonging to the species *Dictamnus* to an extraction with an aqueous solvent, wherein the extraction of the matrine containing plant material is performed separately from the extraction of the plant material derived from a plant belonging to the species *Dictamnus*.

The herbal composition obtained from the present process may advantageously be employed to both prevent and treat various forms of cancer and does not give rise to harmful side effects. Surprisingly it was found that the present composition is not only effective in the treatment of cancer, but also that it can be used to treat or prevent, *H. pylori* infections, chronic inflammatory conditions, cardiovascular diseases or cerebral vascular diseases in mammals.

### DETAILED DESCRIPTION OF THE INVENTION

Accordingly, the present invention is concerned with a process for the manufacture of a herbal composition, said process comprising:
a. subjecting a matrine containing plant material, in the absence of a plant material derived from the species *Dictamnus* to an extraction with an aqueous solvent, optionally followed by a concentration step, to produce a herbal extract I;
b. subjecting a plant material derived from the species *Dictamnus,* preferably in the absence of a matrine containing plant material, to an extraction with an aqueous solvent, optionally followed by a concentration step, to produce a herbal extract II,
c. mixing the herbal extracts I and II;
d. provided the resulting mixture contains a significant amount of water, drying the mixture to a water content of less than 6 wt.%.

The term matrine containing plant material refers to plant materials that contain at least 0.01 % matrine by weight of dry matter. Preferably the matrine containing plant material comprises at least 0.1 % matrine by weight of dry matter.

The term "concentrating" refers to the operation that aims to increase the concentration of the desired component, especially by removing extraction solvent. It should be understood that this term also encompasses the operation of drying an extract so as to remove all or almost all of the aqueous solvent (and endogenous water) contained therein.

The present process offers the advantage that matrine is recovered in substantially higher yields than is the case if the matrine containing plant material is co-extracted with the plant material derived from a plant belonging to the species *Dictamnus.* In general the recovery of martine in the herbal extract I, prior to the optional concentration, exceeds 60%. Preferably said recovery exceeds 80%, more preferably it exceeds 85%.

The amount of matrine in herbal extract I is usually at least 1 wt.% by weight of the dry matter contained in said extract. Preferably said amount of martine is at least 2 wt.% by weight of the dry matter.

In a preferred embodiment of the present process the herbal extract I is concentrated to a water content of less than 50 wt.% before said extract is admixed with the herbal extract II. By first separately concentrating herbal extract I, interactions with components from the *Dictamnus* materialthat would otherwise lead to martine-losses, are prevented

The present process employs an aqueous extraction solvent, i.e. a solvent comprising a significant amount of water, e.g. at least 30 wt.% water, preferably at least 60 wt.%. The aqueous extraction solvent may suitably comprise a co-solvent that is miscible with water. Examples of such co-solvents include ethanol and methanol. Preferably, however, the extractions in steps a. and b. of the present process use water as the extraction solvent.

The extractions in step a. and b. are advantageously carried out at elevated temperatures so as to enhance the efficiency of extraction. Preferably said extractions are conducted at a temperature in excess of 70°C. More preferably the extractions are conducted at a temperature in excess of 85°C. The extractions in step a. and b. are advantageously carried out whilst maintaining the aqueous solvent at boiling point for at least 1 hour, more preferably for 1.5-3 hours.

In a particularly preferred embodiment of the invention, the process additionally comprises a separate step wherein an amount of *Dictamnus*plant material is converted into a granulate, e.g. by grinding or cutting, which granulate is mixed with the herbal extracts I and II , preferably in step c. It was found that by subjecting *Dictamnus* plant material to extraction with an aqueous solvent, not all the active principles contained therein can be extracted effectively. By additionally incorporating some *Dictamnus* plant material in non-extracted ground form, the loss of valuable active principles is avoided. In this embodiment of the present process, preferably 5-70 wt.% of the total amount of *Dictamnus* plant material used in the process is subjected to the extraction in step b. and 30-95 wt.% of said total amount is subjected to a separate processing step to convert it into a granulate.

In order to ensure that a sufficiently high amount of the active principles are extracted during batch extraction from the, preferably finely cut, plant materials, it is preferred to carry out the extraction steps a. and b. with a total (mass) amount of water that exceeds at least 5 times the amount of herb(s). More preferably the total amount of water exceeds at least 10 times the amount of herb(s). The batch extractions may suitable be carried out as two or more successive extractions. It is also possible to employ continuous extraction methods known in the art, e.g. percolation.

During extraction special techniques may be employed to improve the recovery of valuable plant constituents. Such techniques include ultrasonic vibration, enzymatic lysis of the cell walls, etc.

In the present process, prior to step c., the herbal extracts I and II are preferably concentrated by evaporation. Said evaporation is advantageously conducted at reduced pressure and a temperature below 85°C. Thus the degradation of heat labile components is minimised as well as losses of volatile active principles.

In a preferred embodiment of the present process plant material derived from one or more herbs selected from the group consisting of *Polygonum bistorta, Prunella vulgaris, Sonchus brachyotus* and *Dioscorea bulbifera* is co-extracted with the plant material derived from the species *Dictamnus* to produce the herbal extract II and wherein the matrine containing plant material is extracted in the absence of these one or more herbs. The aforementioned herbs contain components that are capable of reinforcing the chemopreventive and therapeutic properties of the components extracted from the matrine containing plant material and the *Dictamnus* plant material. Since co-extraction of these herbs with the matrine containing plant material will negatively influence the recovery of matrine, it is preferred to combine plant material derived from these herbs with the *Dictamnus* plant material to produce herbal extract II.

The botanical. chemical and histological characteristics of the component herbs used in some embodiments of the process of the present invention are described further below.

### Sophora tonkinensis

Scientific/Pharmaceutical name: Radix Sophorae Tonkinensis
Botanical name: Sophora tonkinensis Gapnep. Also known as Sophora subprostrata
Synonyms and Common Names:
   Guang Dou Gen
   Shan- dou gen- (Mandarin)
   Sanzukon (Japanese)
   Santugtin (Korean)
   Vietnamese Sophora root (English)
   Pigeon pea root
   Mountain bean root
   subprostrate
Botanical Family: Leguminosae
Parts used: Dried Roots
Definition of Drug: Vietnamese Sophora Root is the dried root and rhizome of Sophora tonkinensis Gapnep.
Chemical Constituents:
   Matrine
   Oxymatrine
   Anagyrine
   Methylcytisine
   Sophoranone
   Sophoradin
   Sophoranochromente
   Sophoradochromente
   Genistein
   Pterocarpine
   Maackian
   Trifolirhizin
   SitosteroI
   Hexadecanoic acid
Approximately 0.93% of the root of Sophora tonkinensis consists of alkaloids, including matrine, oxymatrine, anagyrine and methylcytisine. The root contains other non-alkaloidal substances, including sophoranone, sophoranochromene, sophoradin and daidzein. Also present is 1-trifolirhizin, a glucoside whose hydrolysed product is 1-maackinin. Other substances isolated from Sophora tonkinensis include pterocarpine and 1- maackain. Also, some triterpenoidal saponins have been isolated from the root: these include sophomeoanodromone and sophomeoadochromone.
Botanical Characteristics :
   General distribution: The herb's distribution in China is in Guangxi, Guizhou and
   Yunnan provinces. The elevation range in China is between 1000-2000m. It is also distributed in Vietnam.
Macroscopic description:
   Rhizomes are irregularly nodiform showing remaining stem-bases at the top and several roots at the lower part. Roots are usually branched and long cylindrical. Its length is variable and its diameter is between 0.7-1.5 cm. The outer part is brown to dull brown with irregular longitudinal wrinkles and protruding transversal lenticels. The texture is hard and tough with brownish fractured surface in bark and pale yellow in wood. Its characteristic odour is similar to bean and its taste is very bitter.
Histology:
   A transverse section demonstrates that the cork layer consists of up to ten or more layers of cells. In the cortex the first and second outer rows of cells contain prisms of calcium oxalate which create a discontinuous ring of crystal cells with lignified and thickened cell walls. Groups of fibres are distributed throughout both the cortex and phloem. The cambium is in a ring. Xylem is well developed, the rays are 1-8 cells wide; vessels are subrounded, mostly single and scattered or in groups of two or more, containing yellowish-brown contents; wood fibres are scattered in groups. Parenchymatous cells are filled with starch grains and a few cells contain prisms.

### Polygonum bistorta

Scientific/Pharmaceutical name: Rhizoma Polygonum Bistorta L.
Botanical name: Polygonum bistorta L.
Common Names:
   Bistort
   Dragonwort
   Easter Giant
   Adderwort
   English Serpentary
   Osterick, Passions
   Patience Dock
   Red Legs
   Sweet Dock
   Snakeweed
   Quánshen (Chinese)
Parts used: Dried rhizomes
Definition of Drug: Bistort root and rhizome is the subterranean part of Polygonum bistorta L.
Chemical Constituents:
   Polyphenols (including ellagic acid)
   Tannins(15-20%)
   Phlobaphene,
   F 1 avonoids
   The anthraquinone emodin.
   Hexadecanoic acid
   Diosgenin
   Iodoquinol
   1 ,4,2-dioxazole,3-(4 chlorphenyl)-5-ph
General distribution:
   Polygonum bistorta is distributed in Europe, Northern and Western Asia; China, Japan, and North America. This perennial grows on slopes, in grassland and under trees; meadows and woodland, and prefers damp conditions.
Macroscopical description:
   Polygonum bistorta is a perennial herb, about 50-80 cm high. Rootstock is crooked, robust, woody and purplish-brown or black, as thick as the thumb or less. The stem is erect, very simple, slender and glabrous. The basal leaves are clustered and long petiolate, oblong-lanceolate, 10-18 cm long by 2.5- 5 cm wide. The apex is acute and the base is obtuse-rounded or truncate, sometimes cordate (heart-shaped) and coriaceous. Sheath of stipules are tube-like, membranous. Cauline leaf is usually linear or lanceolate, sessile or amplexicaul.
   The flowers are reddish or white. Petals are present in numbers of 5 and are elliptic. There are 8 stamens and 3 stigmas. The nut is very small, trigonous, glossy and reddish-brown colored. The herb is odorless but tastes very bitter and harsh.
Histology:
   The transverse section of the rhizoma is pink. The plant's surface when transversely fractured is brownish red and shows vascular bundles arranged in a circle of yellowish white dots.

### Prunella vulgaris

The whole dried plant of Prunena vulgaris is used to produce the extracts of the present invention.
Scientific/Pharmaceutical name: Spica Prunenae Vulgaris
Botanical name: Prunella vulgaris L. (Brunella vulgaris L.)
Synonyms and Common names:
   Self heal,
   Heal all,
   Selfheal spike,
   Prunella,
   "Summer withered herb" (English)
   agos- (Japanese)
   agoch'o (Korean)
   lá kucao (Chinese)
Botanical Family: Labiatae
Parts used: Fruit spike
Definition of Drug:
   The drug, commonly referred to as Selfheal Fruit-Spike, consists of the dried flowered fruit-spike of Prunella vulgaris L.
Chemical constituents:
   Oleanolic acid
   Ursolic acid
   Rutin
   Hyperoside
   Caffeic acid
   Vitamin B1
   Vitamin C
   Vitamin K
   Tannin
   Polysaccharide prunel line
   Triterpenes (two), based on ursolic and oleanolic acid
General distribution:
   Prunella vulgaris is distributed in China (Jiangsu, Anhhui, Zhejiang, Hunan) and Japan.
Macroscopic description:
   The herb is a common weed, a low sprawling perennial about 45 cm high, with a short rhizome, reproducing by seeds and short runners that root freely at the nodes. As with other members of the mint family, the stems are square and the leaves are opposite.
   The stems branch freely and are usually about one foot tall. The lower leaves are petioled with the blades lanceolate to ovate. The upper leaves are sessile. subtending the flower cluster. The flower cluster is a close thick spike with three flowers in the axils of each rounded membranaceous bract. The flowers are two-lipped, the upper lip hood-shaped, the lower lip shorter and three lobed. The blossoms are lavender to white. The herb tastes bitter .

### Sonchus brachyotus

Scientific/Pharmaceutical name: Herba cum Radice Patriniae
Botanical name:
   (in China) Sonchus brachyotus DC. and Sonhus spp.
   (in Japan) Patrinia villosa Juss.
   (in Taiwan and Hong Kong) Thlaspi arvensis L.
Family name:
   Valerianaceae (Japan)
   Cruciferae (Taiwan and Hong Kong)
   Compositae (China)
Synonyms/Common name:
   Pinyin qu mai cai
   Haishoso (Japanese)
   P'aechangch'o (Korean)
   Patrinia thlaspi (English)
   Snow-thistle. Penny-cress. Patrinia.
   Pai-chiang-Tsao. or Bai-Jiang-Cao
Parts used: Dried whole plant
Chemical constituents:
   Thlaspi arvensis (seeds): sinigrin and myrosinde (enzyme)
   S. brachyotus DC (plant): fattyoil, ceryl alcohol, invert sugar, choline, tartaric acid; (juice) oxydase, kautschuk, mannitol. L-inositol, alpha and beta-lactucerol Patrinia villosa (root and rhizome) loganin, viloside, morroniside; (fruit and stem) sinigrin
   Patrinia scabiosaefolia (root and rhizome) oleanolic acid, hederagenin, beta-sitosterol, D-glucoside, saponins (patrinoside A,C, C1,.D, and scabiosides A, B, C, D, E, F, G). ursolic acid
General distribution:
   The China provincial distribution of Sonchus brachyotus DC is in Henongjiang, Jilin, Nei Mongol, Hebei. Shanxi, Shaanxi and Shandong. It is also distributed in Japan, Mongoia and Russia.

### Dictamnus dasycarpus Turcz

Scientific/Pharmaceutical name: Cortex Dictamni Dasycarpi Radicis
Botanical name: Dictamnus dasycarpus Turcz.
Family: Rutaceae
Synonyms/Common names:
   Densefruit Pittany Root-bar
   Bai Xian Pi (Mandarin)
   Hakusenpi (Japanese)
   Paeksonp'i (Korean)
   Cortex of Chinese Dittany Root (Eng.)
   "White Fresh Bark"
Parts used: Dried root bark
Definition of drug:
   Dense Fruit Pittany Root-bark is the dried root bark of Dictamnus dasycarpus Turcz. The root bark is stripped off and dried.
Chemical constituents:
   dictamnine
   skimmianine
   g-fragarine
   preskinnianine and isomaculosinidine
   limonin and obakinone
   fraxinellone
   psoralen
   aurapten and bergapten
   and saponins and essential oils.
General distribution:
   The herb is native to China (Manchuria, Hebei, Henan, Anhui, Jiangsu, and Hubei), and Korea.
Macroscopical description:
   The root bark is quilled, 5-15 cm long, 1-2 cm in diameter, 2-5 mm thick outer surface greyish-white or pale greyish-yellow, with fine longitudinal wrinkles and rootlet scars, frequently with protruding small granular dots. The inner surface is whitish with fine longitudinal wrinkles, smooth, slightly fibrous. The texture is fragile, dusting on breaking, fracture uneven and somewhat lamellar .When the outer layer is peeled off, numerous small glitter spots are observed during exposure to light. Its odor is muttony, and its taste is slightly bitter. The herb also has cold property.

### Dioscorea bulbifera

Pharmaceutical name: Tuber Dioscoreae Bulbiferae
Botanical name: Dioscorea bulbifera L
Family: Dioscoreaceae (625 species)
Synonyms/Common names:
   Bulbil-bearing yam,
   Potato yam,
   Aerial yam,
   Air potato,
   "Yellow medicine "
   Huáng yao zi (Chinese)
   Oyakushi(Japanese)
   Hwangyakcha (Korean)
Parts used: Dried tubers
Chemical constituents of the dried tubers of Dioscorea bulbifera:
   Diosgenin
   Diosbulins A, B, C
   Iodine
   Vitamins A, B, C
   The herb contains a small amount of saponins, including dioscorecin, dioscoretoxin and tannin. Bitter substances include diosbulbine A, B, and C, and iodine.
General distribution:
   The Dioscoreales are primarily a tropical group, with a few species in Europe and temperate America. Most are vines or climbing plants, but some are herbs as well. The Dioscoreales include 600 species of Dioscorea and about 35 species in other genera. The group is not especially large, but the genus Dioscorea is common in wet tropical regions around the world. In many countries, it is cultivated for its starchy tubers, sometimes called air potatoes or Chinese yams.
Macroscopic description:
   A common characteristic of most Dioscoreales is a thick, starchy underground stem called a tuber. The tuber provides an energy store for these plants to begin growth again when conditions are favorable. The tubers of Dioscorea are full of starch and nutrients, and boiled and eaten in many cultures. These provide the primary source of carbohydrates for about 70 million people in Africa and southern Asia. The plants are propagated by cutting up the tubers, rather than from seed, as there are not usually many seeds produced. The Dioscoreales are monocots. The leaves have a reticulated venation reminiscent of the paleoherb dicots, and may be heart-shaped or highly lobed. For this reason, the Dioscoreales have, at times, been considered the most "primitive" of monocots, but though they may retain many characters found in early monocots, they now appear to be more closely related to the Lilliales. The Dioscoreales may be divided into the following families:
      1. Trichopodaceae -Trichopus zeylanicus
      2. Dioscoreaceae -Air potatoes (625 species), mostly Dioscorea
      3. Taccaceae -only Tacca, the "bat flower" , with 10 species.
   Dioscorea bulbifera is covered with large handsome leaves. It has a winter dormant period when the stems die back to ground. After dormancy, the underground tubers give rise to stems which grow quickly, reaching up to 70 feet by the end of the growing season. The vine's stem is herbaceous, not woody. The stem is round, and not winged. The large leaves are up to 8 inches long and heart-shaped (cordate). The leaf blade's basallobes are rounded. Leaf veins radiate from a single point. The leaves have long stems (petioles), and are alternate on the stem.
   Unisexual flowers appear from July to October. They are small, greenish and fragrant, hanging in relatively long clusters (panicles and spikes) up to 4 inches long. The fruit is a capsule of seeds, winged at the base. Dioscorea bulbifera plants produce "aerial tubers" that are attached closely to the stems where leaves attach (axil). These are greyish and somewhat irregular. Tubers also grow underground where they may be much larger, light- yellow to yellowish-brown, and have a bitter taste.
   It is important to ensure that the herbs which are used according to the present invention are selected such that they contain only acceptable levels of contaminants such as metals or pesticides. Various regions of China have been surveyed and it was found that the component herbs are preferably harvested from the Guangxi, Hunan, Liaoning, Anhui, Hebei and Jiangsu regions of China during the summer and autumn months and no pesticides are used. The plants are purchased dried and whole or parts of these herbs are used in mahnufacturing.

In the process according to the invention, the matrine containing plant material is advantageously utilised in an amount of between 5 and 50 %, more preferably of between 10 and 30% by weight of the total amount of plant material utilised in the process. The *Dictamnus* plant material is advantageously utilised in a total amount of between 3 and 30%, more preferably of between 5 and 20% by weight of the total amount of plant materials utilised in the process.

The plant materials derived from the aforementioned herbs *Polygonum bistorta, Prunella vulgaris, Sonchus brachyotus* and *Dioscorea bulbifera* are generally utilised in a total amount of between 30 and 90%, preferably of between 60 and 80% by weight of the total amount of plant materials utilised in the present process.

Step b. of the present process preferably comprises subjecting plant material derived from at least two different herbs to the extraction with an aqueous solvent. Studies into the chemopreventive and therapeutic properties of the herbal compositions obtained by the present process have shown that best results are obtained if plant material derived from two or more, preferably from three or more herbs selected from the group consisting of *Polygonum bistorta, Prunella vulgaris, Sonchus brachyotus* and *Dioscorea bulbifera* is subjected to the extraction with an aqueous solvent in step b. Most preferably step b. comprises extracting plant material derived from the herbs *Polygonum bistorta, Prunella vulgaris, Sonchus brachyotus* and *Dioscorea bulbifera*.

The mixture obtained from step d. of the present process is preferably subjected to further processing steps, including e.g. grinding or spray drying, to produce a pharmaceutical or nutritional product that can suitably be used to administer the active principles contained in the herbal composition. More particularly, the present process advantageously comprises the additional steps of blending the mixture obtained from step d. with excipient material; and converting the resulting blend into a solid unit dosage form for oral administration. In order to avoid that some of the efficacy of the present herbal composition is lost after oral administration as a result of contact with gastric fluids, it is preferred convert the aforementioned blend into a tablet, which tablet is subsequently coated with an enteric coating.

### EXAMPLES

### Example 1

In one illustrative example of the present invention the manufacturing process is as follows: Dictamnus dasycarpus Turcz (105g) is ground into a powder form and set aside for later use. Sophora tonkinensis (420g) is added to ten times its volume in water (approximately 5L) and then set to boil and decoct for 1.5 hours. The fluid is then removed and saved and another ten times volume of water is added. The mixture is boiled and decocted for another 1.5 hours. The fluid is again removed and then combined with the first portion. The solution is filtered to remove large particles and then concentrated by heating at reduced pressure to S.G. of 1.30-1.35 (at 50°C). The resulting cream paste is dehydrated at a temperature under 60°C, pulverised and the Sophora tonkinensis powder is then set aside for later use.

Another portion of Dictamnus dasycarpus Turcz (105g) is mixed with Polygonum bistorta (420g), Prunella vulgaris (420g), Sonchus brachyotus (420g) and Dioscorea bulbifera (100g). The mixture is added to ten times its volume in water (approximately 15L), boiled and then decocted for 2 hours. The fluid is then removed and saved. Another ten times volume of water is added and the boiling and decocting process is repeated. The fluid is again removed and added to the first portion. The solution is filtered to remove large particles and then concentrated at reduced pressure to an S.G. of 1.30-1.35 (50°C) (clear cream paste). The cream paste is then mixed with the Dictamnus dasycarpus powder and the Sophora tonkinensis powder. The mixture is then dehydrated at a temperature below 80°C. The dried herbal extract is then ground to a powder.

An appropriate amount of starch and a small amount of ethanol is added as binding material and 0.3g tablets are formed. Sugar is then used to coat the tablets. The recipe produces about 1,000 tablets.

Thus one example of the present invention is a combination of Sophora tonkinensis, Polygonum bistorta, Prunella vulgaris, Sonchus brachyotus, Dictamnus dasycarpus Turcz, and Dioscorea bulbifera in an amount of about 21%,21%,21%,21%, 10.5%, and 5.5%, respectively.

### Comparative example

Example 1 is repeated with the following exception: The Sophora tonkinensis (420g) is mixed with Dictamnus dasycarpus Turcz (105g), Polygonum bistorta (420g), Prunella vulgaris (420g), Sonchus brachyotus (420g) and Dioscorea bulbifera (100g). The mixture is added to ten times its volume in water (approximately 20L), boiled and then decocted for 2 hours. The fluid is then removed and saved. Another ten times volume of water is added and the boiling and decocting process is repeated.

The matrine recovery in the extract so obtained is found to be much lower than what is observed for the extract obtained by separate extraction of Sophora tonkinensis.

### Example 2

In a further embodiment of the present invention Herb F is reduced by about 75%, because certain patients taking the herbal composition of Example 1, showed eleyated liver enzymes. Thus, according to this embodiment of the present invention, the formulation comprises a lower concentration of the Dioscorea bulbifera . In one example of this embodiment there is provided a composition comprising a combination of Sophora tonkinensis, Polygonum bistorta, Prunella vulgaris, Sonchus brachyotus, Dictamnus dasycarpus Turcz, and Dioscorea bulbifera in an amount of about 21.9%, 21.9%, 21.9%, 21.9%, 11.0%, 1.4%.

### Example 3

In yet a further embodiment of the present invention Dioscorea bulbifera is eliminated entirely from the composition. Thus, according to this embodiment the composition comprises a combination of Sophora tonkinensis, Polygonum bistorta, Prunella vulgaris, Sonchus brachyotus and Dictamnus dasycarpus Turcz in an amount of from 6%-38%, 6%-38%, 6%-38%, 6%-38%, and 3%-19%, respectively. In one specific example of this embodiment there is provided a composition comprising a combination of Sophora tonkinensis, Polygonum bistorta, Prunella vulgaris, Sonchus brachyotus and Dictamnus dasycarpus Turcz in an amount of about 22%, 22%, 22%, 22%, and 11 %, respectively.

## Claims

1. A process for the manufacture of a herbal composition, said process comprising:
a. subjecting a matrine containing plant material, in the absence of a plant material derived from the species *Dictamnus,* to an extraction with an aqueous solvent, optionally followed by a concentration step, to produce a herbal extract I;
b. subjecting a plant material derived from the species *Dictamnus*, to an extraction with an aqueous solvent, optionally followed by a concentration step, to produce a herbal extract II;
c. mixing the herbal extracts I and II;
d. provided the resulting mixture contains a significant amount of water, drying the mixture to a water content of less than 6 wt.%.

2. The process according to claim 1, wherein the recovery of matrine in herbal extract I, prior to the optional concentration, exceeds 85%.

3. The process according to claim 1 or 2, wherein, prior to the admixture with herbal extract II, the herbal extract I is dried to a water content of less than 50 wt.%.

4. The process according to any one of claims 1-3, wherein the extractions in steps a. and b. use water as the extraction solvent.

5. The process according to any one of claims 1-4, wherein the extractions in step a. and b. are carried out at a temperature in excess of 70°C.

6. The process according to any one of claims 1-5, wherein the extractions in step a. and b. are carried out whilst maintaining the aqueous solvent at boiling point for at least 1 hour.

7. The process according to any one of claims 1-6, wherein the process additionally comprises a separate step wherein an amount of a plant material derived from the species *Dictamnus* is converted into a granulate which is mixed with the herbal extracts I and II.

8. The process according to claim 7, wherein 5-70 wt.% of the total amount of the plant material derived from the species *Dictamnus*that is used in the process, is subjected to the extraction in step b. and 30-95 wt.% of said total amount is subjected to a separate processing step to convert it into granulate.

9. The process according to any one of claims 1-8, wherein plant material derived from one or more herbs selected from the group consisting of *Polygonum bistorta, Prunella vulgaris, Sonchus brachyotus* and *Dioscorea bulbifera* is co-extracted with the plant material derived from the species *Dictamnus* to produce the herbal extract II and wherein the matrine containing plant material is extracted in the absence of these one or more herbs.

10. The process according to any one of claims 1-9, wherein the extractions in steps a. and b. are carried out with a total amount of water that exceeds at least 5 times the amount of herb(s).

11. The process according to any one of claims 1-10, wherein, prior to step c., the herbal extracts I and II are concentrated by evaporation at reduced pressure and a temperature below 85°C.

12. The process according to any one of claims 1-11, wherein the matrine containing plant material is utilised in an amount between 5 and 50 % by weight of the total amount of plant materials utilised in the process.

13. The process according to any one of claims 1-12, wherein herb plant material derived from the species *Dictamnus* is utilised in a total amount of between 3 and 30% by weight of the total amount of plant materials utilised in the process.

14. The process according to any one of claims 1-13, wherein matrine containing plant material is derived from *Sophora tonkinensis*.

15. The process according to any one of claims 1-14 wherein the plant material derived from the species *Dictamnus* is *Dictamnus dasycarpus*.

16. The process according to any one of claims 1-15, wherein step b. comprises subjecting plant material derived from at least two different herbs to the extraction with an aqueous solvent.

17. The process according to any one of claims 1-16, wherein the process comprises the additional steps of
f. blending the mixture obtained from step d. with excipient material; and
g. converting the resulting blend into a solid unit dosage form for oral administration.

18. The process according to claim 17, wherein the step g. comprises converting the resulting blend into a tablet, which tablet is subsequently coated with an enteric coating.

## Patentansprüche

1. Verfahren zur Herstellung einer Kräuterpflanzenzusammensetzung, umfassend:
a) Extraktion eines Matrin enthaltenden Pflanzenmaterials mit einem wäßrigen Lösemittel in Abwesenheit eines von der Spezies *Dictamnus* stammenden Pflanzenmaterials, gegebenenfalls gefolgt von einem Konzentrationsschritt, um ein Kräuterpflanzenextrakt I herzustellen;
b) Extraktion eines von der Spezies *Dictamnus* stammenden Pflanzenmaterials mit einem wäßrigen Lösemittel, gegebenenfalls gefolgt von einem Konzentrationsschritt, um ein Kräuterpflanzenextrakt II herzustellen;
c) Mischen der Kräuterpflanzenextrakte I und II;
d) Trocknung der Mischung auf einen Wassergehalt von weniger als 6 Gew.-%, sofern die resultierende Mischung einen signifikanten Wassergehalt aufweist.

2. Verfahren nach Anspruch 1, wobei die Rückgewinnung bzw. Ausbeute von Matrin im Kräuterpflanzenextrakt I vor der optionalen Konzentrierung 85 % übersteigt.

3. Verfahren nach Anspruch 1 oder 2, wobei vor der Beimischung des Kräuterpflanzenextrakts II das Kräuterpflanzenextrakt I auf einen Wassergehalt von weniger als 50 Gew.-% getrocknet wird.

4. Verfahren nach einem der Ansprüche 1 bis 3, wobei die Extraktionen in Schritt a) und b) Wasser als Extraktionslösemittel verwenden.

5. Verfahren nach einem der Ansprüche 1 bis 4, wobei die Extraktionen in Schritt a) und b) bei einer Temperatur von mehr als 70 C° durchgeführt werden.

6. Verfahren nach einem der Ansprüche 1 bis 5, wobei die Extraktionen in Schritt a) und b) durchgeführt werden, während das wäßrige Lösemittel für mindestens eine Stunde lang bei Siedetemperatur belassen wird.

7. Verfahren nach einem der Ansprüche 1 bis 6, wobei das Verfahren zusätzlich einen separaten Schritt umfaßt, wobei eine Menge eines von der Spezies *Dictamnus* stammenden Pflanzenmaterials in ein Granulat, welches mit den Kräuterpflanzenextrakten I und II gemischt wird, überführt wird.

8. Verfahren nach Anspruch 7, wobei 5 bis 70 Gew.-% der Gesamtmenge des in dem Verfahren verwendeten, von der Spezies *Dictamnus* stammenden Pflanzenmaterials der Extraktion in Schritt b) unterzogen und 30 bis 95 Gew.-% der Gesamtmenge einem separaten Verfahrensschritt zu dessen Umwandlung in ein Granulat unterworfen werden.

9. Verfahren nach einem der Ansprüche 1 bis 8, wobei das Pflanzenmaterial, welches von mindestens einer Kräuterpflanze, ausgewählt aus der Gruppe von *Polygonum bistorta, Prunella vulgaris, Sonchus brachyotus* und *Dioscorea bulbifera*, stammt, mit dem von der Spezies *Dictamnus* stammenden Pflanzenmaterial zur Herstellung des Kräuterextrakts II co-extrahiert wird und wobei das Matrin enthaltende Pflanzenmaterial in Abwesenheit der mindestens einen Kräuterpflanze extrahiert wird.

10. Verfahren nach einem der Ansprüche 1 bis 9, wobei die Extraktionen in Schritt a) und b) mit einer Gesamtmenge an Wasser durchgeführt wird, welche mindestens das 5fache der Menge an Kräuterpflanze(n) überschreitet.

11. Verfahren nach einem der Ansprüche 1 bis 10, wobei vor Schritt c) die Kräuterpflanzenextrakte I und II mittels Verdampfen bei reduziertem Druck und einer Temperatur von weniger als 85 °C konzentriert werden.

12. Verfahren nach einem der Ansprüche 1 bis 11, wobei das Matrin enthaltende Pflanzenmaterial in einer Menge von 5 bis 50 Gew.-%, bezogen auf die Gesamtmenge des in dem Verfahren verwendeten Pflanzenmaterials, eingesetzt wird.

13. Verfahren nach einem der Ansprüche 1 bis 12, wobei das von der Spezies *Dictamnus* stammende Kräuterpflanzenmaterial in einer Menge von 3 bis 30 Gew.-%, bezogen auf die Gesamtmenge des in dem Verfahren verwendeten an Pflanzenmaterials, eingesetzt wird.

14. Verfahren nach einem der Ansprüche 1 bis 13, wobei das Matrin enthaltende Pflanzenmaterial von *Sophora tonkinensis* stammt.

15. Verfahren nach einem der Ansprüche 1 bis 14, wobei das von der Spezies *Dictamnus* stammende Pflanzenmaterial von *Dictamnus dasycarpus* stammt.

16. Verfahren nach einem der Ansprüche 1 bis 15, wobei Schritt b) die Extraktion von Pflanzenmaterial, welches von mindestens zwei verschiedenen Kräuterpflanzen stammt, mit einem wäßrigen Lösemittel umfaßt.

17. Verfahren nach einem der Ansprüche 1 bis 16, wobei das Verfahren die zusätzlichen Schritte
f) des Mischens der in Schritt d) erhaltenen Mischung mit Arzneiträgermaterial bzw. Exzipienten; und
g) der Weiterverarbeitung der resultierenden Mischung zu einer festen Dosierungseinheit zur oralen Applikation
umfaßt.

18. Verfahren nach Anspruch 17, wobei Schritt g) die Weiterverarbeitung der resultierenden Mischung zu einer Tablette umfaßt, wobei die Tablette nachfolgend mit einer darmlöschlichen bzw. magensaftresistenten Beschichtigung beschichtet wird.

## Revendications

1. Procédé de fabrication d'une composition de herbe médicinale, ledit procédé comprenant de :
a. soumettre un matériau végétal contenant de la matrine, en absence d'un matériau végétal dérivé de l'espèce *Dictamnus,* à une extraction avec un solvant aqueux, éventuellement suivie par une étape de concentration, pour produire un extrait de herbe médicinale I ;
b. soumettre un matériau végétal dérivé de l'espèce *Dictamnus,* à une extraction avec un solvant aqueux, éventuellement suivie par une étape de concentration, pour produire un extrait de herbe médicinale II ;
c. mélanger les extraits de herbe médicinale I et II ;
d. si le mélange obtenu contient une quantité importante d'eau, sécher le mélange jusqu'à une teneur en eau de moins de 6 % en poids.

2. Procédé selon la revendication 1, dans lequel la récupération de la matrine dans l'extrait de herbe médicinale I, avant la concentration éventuelle, dépasse 85 %.

3. Procédé selon l'une des revendications 1 ou 2, dans lequel, avant le mélange avec l'extrait de herbe médicinale II, l'extrait de herbe médicinale I est séché jusqu'à une teneur en eau de moins de 50 % en poids.

4. Procédé selon l'une quelconque des revendications 1 à 3, dans lequel les extractions dans les étapes a. et b. utilisent l'eau comme solvant d'extraction.

5. Procédé selon l'une quelconque des revendications 1 à 4, dans lequel les extractions dans l'étape a. et b. sont effectuées à une température dépassant 70°C.

6. Procédé selon l'une quelconque des revendications 1 à 5, dans lequel les extractions dans l'étape a. et b. sont effectuées tout en maintenant le solvant aqueux au point d'ébullition durant au moins 1 heure.

7. Procédé selon l'une quelconque des revendications 1 à 6, dans lequel le procédé comprend de plus une étape distincte dans laquelle une quantité d'un matériau végétal dérivé de l'espèce *Dictamnus* est convertie en granulés qui sont mélangés avec les extraits de herbe médicinale I et II.

8. Procédé selon la revendication 7, dans lequel 5 à 70 % en poids de la quantité totale du matériau végétal dérivé de l'espèce *Dictamnus* qui sont utilisés dans le procédé, sont soumis à l'extraction dans l'étape b., et 30 à 95 % en poids de ladite quantité totale sont soumis à une étape de traitement distincte pour les convertir en granulés.

9. Procédé selon l'une quelconque des revendications 1 à 8, dans lequel le matériau végétal dérivé d'une ou plusieurs herbes médicinales choisies dans le groupe constitué par *Polygonum bistoria, Prunella vulgaris, Sonchus brachyotus* et *Dioscorea bulbifera* est co-extrait avec le matériau végétal dérivé de l'espèce *Dictamnus* pour produire l'extrait de herbe médicinale II, et dans lequel le matériau végétal contenant de la matrine est extrait en absence de ces une ou plusieurs herbes médicinales.

10. Procédé selon l'une quelconque des revendications 1 à 9, dans lequel les extractions dans les étapes a. et b. sont effectuées avec une quantité d'eau totale qui dépasse d'au moins 5 fois la quantité de herbe(s) médicinale(s).

11. Procédé selon l'une quelconque des revendications 1 à 10, dans lequel, avant l'étape c., les extraits de herbe médicinale I et II sont concentrés par évaporation à pression réduite et à une température au-dessous de 85°C.

12. Procédé selon l'une quelconque des revendications 1 à 11, dans lequel le matériau végétal contenant de la matrine est utilisé en une quantité entre 5 et 50 % en poids de la quantité totale de matériaux végétaux utilisés dans le procédé.

13. Procédé selon l'une quelconque des revendications 1 à 12, dans lequel le matériau végétal de herbe médicinale dérivé de l'espèce *Dictamnus* est utilisé en une quantité totale entre 3 et 30 % en poids de la quantité totale de matériaux végétaux utilisés dans le procédé.

14. Procédé selon l'une quelconque des revendications 1 à 13, dans lequel le matériau végétal contenant de la matrine est dérivé de *Sophora tonkinensis*.

15. Procédé selon l'une quelconque des revendications 1 à 14, dans lequel le matériau végétal dérivé de l'espèce *Dictamnus* est *Dictamnus dasycarpus*.

16. Procédé selon l'une quelconque des revendications 1 à 15, dans lequel l'étape b. comprend de soumettre un matériau végétal dérivé d'au moins deux herbes médicinales différentes à l'extraction avec un solvant aqueux.

17. Procédé selon l'une quelconque des revendications 1 à 16, dans lequel le procédé comprend les étapes supplémentaires de
f. mélanger le mélange obtenu dans l'étape d. avec un matériau excipient ; et
g. convertir le mélange obtenu en une forme posologique unitaire solide pour une administration orale.

18. Procédé selon revendication 17, dans lequel l'étape g. comprend de convertir le mélange obtenu en un comprimé, lequel comprimé est ensuite enrobé avec un enrobage entérique.
